(19) Europäisches Patentamt
European Patent Office
Office européen des brevets

(11) Publication number: 0 245 680 A2

(12) # EUROPEAN PATENT APPLICATION

(21) Application number: 87106044.8

(22) Date of filing: 24.04.87

(51) Int. Cl.4: C07D 211/90 , //A61K31/44

(30) Priority: 05.05.86 IT 2030586

(43) Date of publication of application: 19.11.87 Bulletin 87/47

(84) Designated Contracting States: AT BE CH DE ES FR GB GR IT LI LU NL SE

(71) Applicant: ISTITUTO LUSO FARMACO D'ITALIA S.p.A.
Via Carnia 26
I-20132 Milano(IT)

(72) Inventor: Salimbeni, Aldo
Via Carnia, 26
I-20132 Milano(IT)
Inventor: Manghisi, Elso
Via Carnia, 26
I-20132 Milano(IT)

(74) Representative: Minoja, Fabrizio
Studio Consulenza Brevettuale Via Rossini, 8
I-20122 Milano(IT)

(54) **A process for the preparation of 2-(N-benzyl-N-methylamino)-ethyl-methyl-1,4-dihydro-2,6-dimethyl-4-(3-nitrophenyl)-3,5-pyridine dicarboxylate hydrochloride.**

(57) 2-(N-benzyl-N-methylamino)-ethyl-methyl 1,4-dihydro-2,6-dimethyl-4-(3-nitrophenil)-3,5-pyridine-dicarboxylate hydrochloride is prepared by reation of 3-nitro-benzaldehyde, methyl 3-aminocrotonate and 2-(N-methylamino)ethyl acetoacetate and subsequent reaction with a benzyl halide in inert solvents.

EP 0 245 680 A2

# A PROCESS FOR THE PREPARATION OF 2-(N-BENZYL-N-METHYLAMINO)-ETHYL-METHYL-1,4-DIHYDRO-2,6-DIMETHYL-4-(3-NITROPHENYL)-3,5-PYRIDINE DICARBOXYLATE HYDROCHLORIDE.

The present invention relates to a process for the preparation of 2-(N-benzyl-N-methylamino)-ethyl-methyl-1,4-dihydro-2,6-dimethyl-4-(3-nitrophenyl)-3,5-pyridine-dicarboxylate hydrochloride of formula I

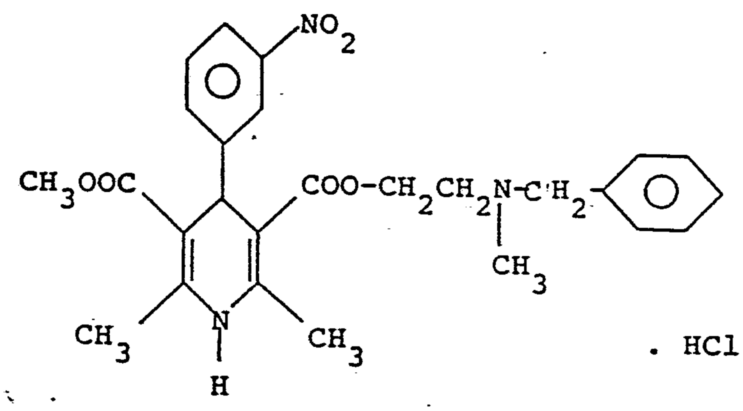

(I)

Said compound, which is known as "Nicardipine", is commonly used in human therapy as a cerebral vasodilator, but it proved to be also effective for the treatment of angina and hypertension. The known processes for the preparation of compound I are described for instance in Chem. Pharm. Bull. 27, 1426 (1979) and may be summarized as follows:

a) reaction of 3-nitrobenzaldehyde with 2-(N-benzyl-N-methyl-amino)ethyl acetoacetate and methyl 3-aminocrotonate;

b) reaction of 2-(N-benzyl-N-methylamino)ethyl 2-(3-nitrobenzylidene)acetoacetate and methyl 3-aminocrotonate;

c) reaction of methyl 2-(3-nitrobenzylidene)aceto acetate and 2-(N-benzyl-N-methylamino)ethyl 3-aminocrotonate;

d) reaction of 2-(N-benzyl-N-methylamino)ethyl 2-(3-nitrobenzylidene)acetoacetate and methyl acetoacetate in the presence of $NH_3$.

Now it has surprisingly been found an improved process for the preparation of compound I in higher yields and purity than those hitherto obtained by means of the known processes.

The method according to the present invention is illustrated in the following scheme:

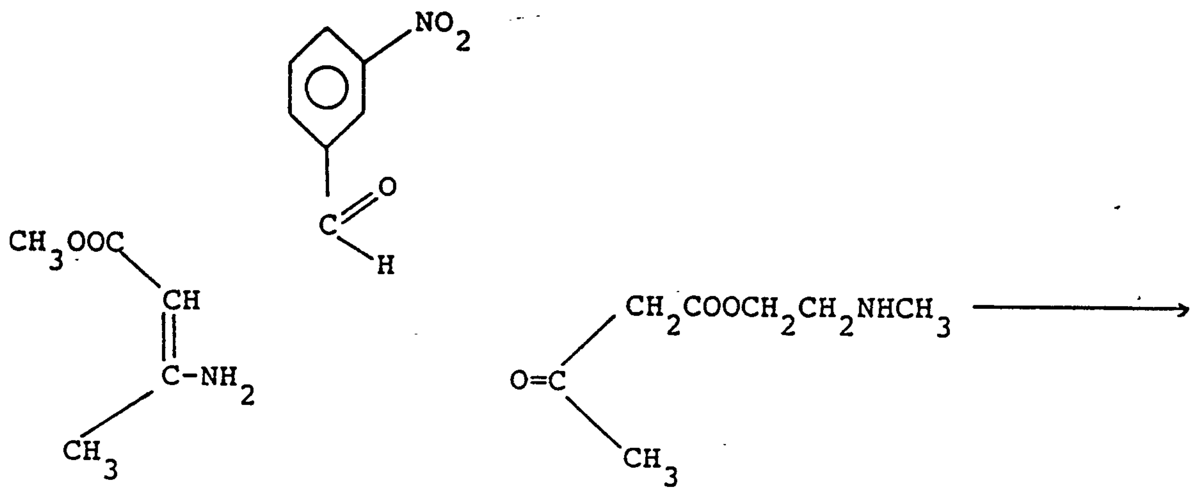

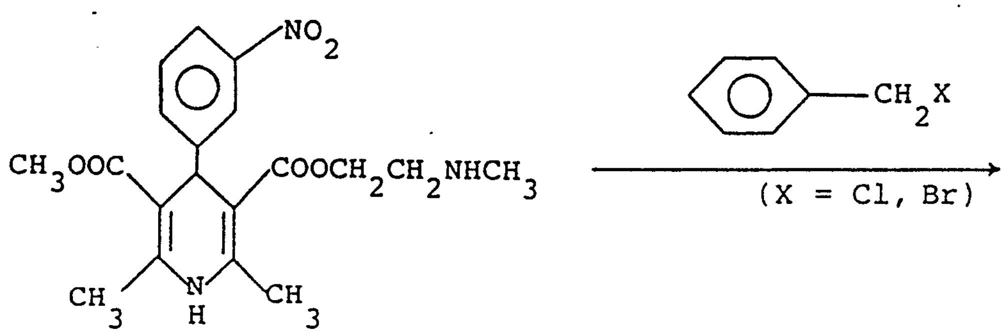


(I)

(II)

According to the process of the invention, 3-nitrobenzaldehyde, methyl 3-aminocrotonate and 2-(N-methylami no)ethyl acetoacetate are reacted in equimolecular amounts, preferably in hydroxylated solvents (such as methanol, ethanol, isopropanol, etc.) or in dipolar aprotic solvents (such as DMF, DMSO, acetonitrile, etc.) at a temperature which may vary from 20° to 150°C, preferably at the solvent's boiling temperature. The reaction time may range from 4 to 12 hours, depending on the used solvent.

When the condensation reaction is complete, solvent is removed and the oily residue, i.e. methyl 2-(N-methylamino)ethyl 1,4-dihydro-2,6-dimethyl-4-(3-nitrophenyl)-3,5-pyridine-dicarboxylate (II), is dissolved in an apolar aprotic solvent (such as benzene, toluene, xylene, and the like), then reacted with an equimolecular amount of a benzyl halide (benzyl bromide, for example) in the presence of a base, such as an alkali carbonate or bicarbonate ($Na_2CO_3$, $K_2CO_3$ etc.) at the solvent's boiling temperature, for a time varying from 6 to 24 hours.

The so obtained compound I (nicardipine) is separated form the reaction mixture by means of a conventional working-up, the purified by crystallization.

The structure of the substance is ascertained by means of elemental analysis and IR, NMR and UV spectra.

The following non-limiting example further illustrates the process of the invention.

## Example

A solution of 10.7 g of 3-nitrobenzaldehyde, 11.3 g of 2-(N-methylamino)ethyl acetoacetate and 8.2 g of methyl 3-aminocrotonate in 50 ml of isopropanol was refluxed for 6 hours.

Solvent was removed under vacuum and the residual oil was dissolved in 300 ml of toluene. 9.8 g of $K_2CO_3$ and 9 g of benzyl chloride were added to the mixture, which was refluxed for 12 hours.

After cooling, the solid was filtered and the toluene solution was washed with $H_2O$. The solvent was removed under vacuum, the residue was-dissolved in $CHCl_3$ and the obtained solution was repeatedly washed with diluted HCl and $H_2O$.

After drying over anhydrous sodium sulfate, the organic phase was evaporated to dryness and the residue was dissolved in ethyl acetate and stirred at a temperature lower than 5°C until solidification.

After filtration and crystallization from acetone, 24.9 g of a yellow crystalline solid were obtained, melting at 166-168°C.

## Claims

1. A process for the preparation of 2-(N-benzyl-N-methyl-amino)-ethyl-methyl 1,4-dihydro-2,6-dimethyl-4-(3-nitrophenyl)-3,5-pyridine-dicarboxylate hydrochloride of formula I

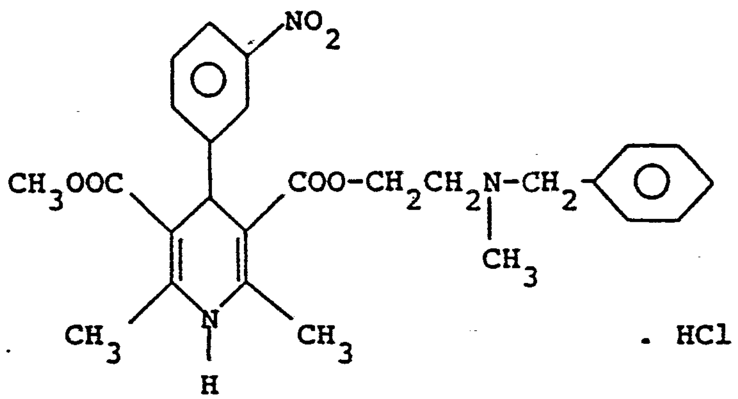

(I)

in which process 3-nitrobenzaldehyde, 2-(N-methylamino-ethyl acetoacetate and methyl 3-aminocrotonate are reacted in substantially equimolecular ratios, and the obtained compound is reacted with a benzyl halide.

2. A process as claimed in claim 1, wherein the reaction between 3-nitrobenzaldehyde, 2-(N-methylamino)ethyl acetoacetate and methyl 3-aminocrotonate is carried out in hydroxilated solvents such as methanol, ethanol or isopropanol.

3. A process as claimed in claim 2, wherein the reaction is carried out at the solvent's boiling temperature.

4. A process as claimed in claim 1, wherein the benzyl halide is benzyl chloride.

5. A process as claimed in claim 1, wherein N-benzyla tion is carried out in apolar aprotic solvents such as benzene, toluene, xylene.

6. A process as claimed in claim 5, wherein the reaction is carried out in the presence of a base, such as an alkali carbonate or bicarbonate.

7. A process as claimed in claim 5, wherein the reation is carried out at a temperature between 80 and 140°C.

8. A process for the preparation of 2-(N-benzyl-N-methyl-amino)-ethyl-methyl 1,4-dihydro-2,6-dimethyl-4-(3-nitrophenyl)-3,5-pyridine-dicarboxylate hydrochloride in which process compound of formula II

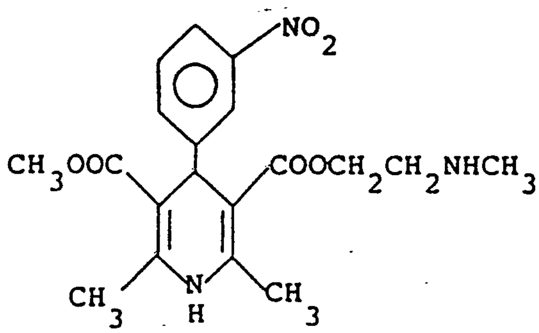

(II)

is reacted with a benzyl halide.

9. A process as claimed in claim 8, wherein the reaction is carried out in apolar aprotic solvents, in the presence of alkali carbonates or bicarbonates.